# EUROPÄISCHE PATENTSCHRIFT

(11) **EP 0 387 324 B1**
(45) Veröffentlichungstag und Bekanntmachung des Hinweises auf die Patenterteilung: **22.11.1995**
(21) Anmeldenummer: 89909391.8
(22) Anmeldetag: 18.08.1989
(51) Int. Cl.: A61F 9/00, A61B 17/36

(54) **VORRICHTUNG ZUR MEDIZINISCHEN CHIRURGIE VON BIOLOGISCHEM GEWEBE MITTELS EINES LASERSTRAHLS**
DEVICE FOR PERFORMING LASER SURGERY ON BIOLOGICAL TISSUE
DISPOSITIF POUR PRATIQUER DE LA CHIRURGIE AU LASER SUR DES TISSUS BIOLOGIQUES

(30) Priorität: 13.09.1988 DE 3831141
(43) Veröffentlichungstag der Anmeldung: 19.09.1990
(73) Patentinhaber: Carl Zeiss, D-73446 Oberkochen (DE); CARL ZEISS-STIFTUNG HANDELND ALS CARL ZEISS, D-89518 Heidenheim (Brenz) (DE)
(72) Erfinder: MÜLLER, Gerhard, D-1000 Berlin 38 (DE); MÜLLER-STOLZENBURG, Norbert, D-1000 Berlin 45 (DE)
(86) Internationale Anmeldenummer: EP8900973
(87) Internationale Veröffentlichungsnummer: WO9002537

(56) Entgegenhaltungen:
- EP-A- 0 266 038
- WO-A-87/01273
- WO-A-88/07841
- US-A- 3 900 034
- US-A- 4 428 748
- Francis A. L'Esperance - Ophthalmic Lasers - 1983 The C.V. Mosby Company, St. Louis, Toronto, London, seiten 340-345
- IEEE Journal of Quantum Electronics, Band QE-23, Nr. 10, Oktober 1987 Canadian Crown New York, NY (US) D.L. SINGLETON et al.: "Excimer Laser Angioplasty: Tissue Ablation, Arteria Response and Fiber Optic Delivery", seiten 1772-1781 siehe Bezeichung und Zusammenfassung
- "An Ultrastructural Study of Corneal Incisions Induced by an Excimer Laser at 193 nm" by J.Marshall et al;Ophthalmology 1985,12 p.749-758.

## Beschreibung

Es ist bereits verschiedentlich vorgeschlagen worden, biologisches Gewebe unter Ausnutzung des bekannten Effektes der Photodekomposition (Photoätzen) mittels kurzer Laserimpulse abzutragen. Entsprechende Veröffentlichungen finden sich in
1. Health Physics Vol. 40, 1981, s. 677-683, Taboda et al: "Response of the corneal Epithelium to KrF Excimer Laser Pulses";
2. American Journal of Ophthalmology 96, 1983 S. 710-715, Trokel et al: "Excimer surgery of the cornea"
3. Ophthalmology 92, 1985, S. 741-748, Puliafito et al: "Excimerlaser ablation of the cornea and lens";
4. Arch. Ophthalmology 103, 1985, S. 1741/1742, Krueger und Trokel:
   "Quantitation of corneal ablation by ultraviolet laser light";
5. Ophthalmology 12, 1985, S. 749-758, Marshall et al: "An ultrastructural study of corneal incisions induced by an excimerlaser at 193 nm; (siehe Oberbegriff von Anspruch 1)
6. American Journal of Ophthalmology Vol. 103, S. 713/714, Berlin et al:
   "Excimerlaser Photoablation in Glaukoma Filtering Surgery;
7. American Journal of Ophthalmology Vol. 99, S. 483,484, Pellin et al:
   "Endoexcimerlaser Intraocular Ablative Photodecomposition";
8. Arch. Ophthalmology Vol. 104, 1986, S. 1825-1829 Nanevicz: "Excimerlaser ablation of the lens".
9. WO-A-8 807 841
10. "Ophthalmic Lasers" von F.A. L'Esperance, C.V. Mosby Company, St. Louis, Toronto, London, 1983

Die Gewebeabtragung mit Pulslasern am Auge ist außerdem Gegenstand der US-Patente 46 86 979 und 47 44 360.

In den genannten Veröffentlichungen und Patenten ist beschrieben, wie man unter Verwendung von Excimer-Lasern unter anderem der Wellenlänge 193 nm und 308 nm Corneagewebe, Linsenkerne, Skleragewebe im Kammerwinkel, Glaskörpergewebe oder Gewebe des Augenhintergrundes nach der Methode der Photoablation bei minimaler thermischer Nekrose der Schnittränder abtragen kann. Mit Excimer-Lasern in diesem Wellenlängenbereich sind feinere Schnitte möglich als mit Nd-YAG oder CO2-Lasern im Wellenlängenbereich zwischen 1 µm und 10 µm, nicht nur aufgrund der kürzeren Wellenlänge der Excimer-Laser sondern aufgrund des grundsätzlich anderen Wirkungsprozesses der Photodekomposition, der wie in den Veröffentlichtungen beschrieben ein Schwellenprozess ist und in Abhängigkeit von der benutzen Wirkwellenlänge des Lasers erst oberhalb bestimmter Energiedichten stattfindet.

Bei der medizinischen Chirurgie von biologischem Gewebe mittels Laserstrahlung tritt immer die Schwierigkeit auf die Wellenlänge der Laserstrahlung so zu wählen, daß sie mit den Absorptionseigenschaften des Gewebes weitgehend übereinstimmt. Ist diese Übereinstimmung nicht gegeben, so ist die zur Gewebeabtragung erforderliche Energiedichte der Laserstrahlung hoch zu wählen, woraus sich relativ große thermische Nekrose-Zonen entlang der Schnittränder ergeben.

Wegen dieser Schwierigkeiten geht die Tendenz dahin, für chirurgische Eingriffe an unterschiedlichen biologischem Gewebe verschiedene Laser einzusetzen, bzw. mit unterschiedlichen Wellenlängen zu arbeiten. Dies ist mit einem relativ großen Aufwand verbunden.

Im Dokument WO-A-8 807 841 (Veröffentlicht zwischem dem Anmeldetag und dem Prioritätstag des vorliegenden Patents) ist eine Vorrichtung zur medizinischen Chirurgie von biologischem Gewebe mittels eines Laserstrahls beschrieben, bei der dem Gewebe eine im Bereich der Wellenlänge des Laserstrahls absorbierende Substanz zugeführt wird. Eine derartige Vorrichtung und ihre Anwendung zur Mikrochirurgie am Auge ist ebenfalls in dem Buch "Ophthalmic Lasers" von F.A. L'Esperance beschrieben.

Der Erfindung liegt nun die Aufgabe zugrunde eine Vorrichtung der vorbekannten Art zur medizinischen Chirurgie von biologischem Gewebe mittels Laserstrahlung dahingehend zu verbessern, daß die Gefahr einer Schädigung gesunden Gewebes weiter minimiert wird.

Diese Aufgabe wird gemäß den im Kennzeichen des Anspruches 1 angegebenen Merkmale dadurch gelöst, daß in der Vorrichtung eine Elektronikeinheit vorgesehen ist, welche die Zugabe der absorbierenden Substanz in der Zeit zwischen den Laserpulsen bewirkt.

Die Erfindung macht von der Erkenntnis Gebrauch, daß es möglich ist, die zu entfernenden biologischen Materialien mit Substanzen die im Bereich der Laserwellenlänge absorbieren, so einzufärben, daß weitgehend unabhängig von den Absorbtionseigenschaften des Gewebes selbst zum einen die Wirkschwelle für den Prozeß der Abtragung deutlich absinkt und zum anderen, das ablatierte Volumen pro applizierten Laserpuls deutlich ansteigt. Gleichzeitig werden thermische Nekrosezonen an den Schnitträndern drastisch reduziert. Dies ist eine Folge geringerer Temperaturerhöhung im Gewebe aufgrund des effektiveren Abtragungsprozesses, wenn vor bzw. während der Laserbehandlung eine absorbierende Substanz zugegeben ist.

Die Vorrichtung nach der Erfindung findet besonders vorteilhaft Anwendung zur Mikrochirurgie am Auge. Dabei wird Laserstrahlung verwendet, deren Wellenlänge im ultravioletten Spektralbereich liegt, beispielsweise bei 308 nm oder 193 nm. Die Gewebeabtragung findet hierbei nach der Methode der Photoablation statt. Die dabei auftretende Photodekomposition ist ein Schwellenprozeß und findet in Abhängigkeit von der benutzten Wellenlänge der Laserstrahlung erst oberhalb einer bestimmten Energiedichte, der sogenannten Wirkschwelle statt. Durch die Zugabe von Substanzen, die im Wellenlängenbereich der Laserstrahung absorbieren, läßt sich zum einen die Wirkschwelle deutlich absenken, zum anderen reduziert die zugegebene Substanz vagabundierende Streustrahlung und Fluoreszenzstrahlung im Auge so, daß die Gefahr einer Kataraktbildung bzw. einer Retinaschädigung minimiert ist.

Wird bei einer Wellenlänge von 308 nm gearbeitet, so besteht das Risiko einer Kataraktinduktion, wenn im vorderen Augenabschnitt gearbeitet wird, bzw. von Retinaschäden, wenn im Glaskörper gearbeitet bzw. die Augenlinse entfernt wird, da mit Ausnahme der Linse die Augenmedien für die Wellenlänge 308 nm in hohem Maße durchlässig sind. Die Zugabe einer bei 308 nm absorbierenden Substanz reduziert die bei der Behandlung auftretende vagabundierende Streustrahlung der Laserwellenlänge im Auge, d.h. das Laserlicht wird von der Linse bzw. der Retina ferngehalten, so daß die Gefahr einer Schädigung weitestgehend minimiert ist.

Wird bei einer Wellenlänge von 193 nm gearbeitet, so muß der Nachteil in Kauf genommen werden, daß es im gegenwärtigen Zeitpunkt keine Lichtleitfaser zur Übertragung der Strahlung auf die Operationsstelle gibt. Eingriffe im Augeninneren sind deshalb nicht ohne weiteres möglich. Die Verwendung der Wellenlänge 193 nm bringt aber gegenüber der Wellenlänge von 308 nm den Vorteil mit sich, daß die thermischen Nekrosezonen um den eigentlich abzutragenden Bereich herum um den Faktor 20 - 50 mal kleiner gehalten werden können.

Bisher hat man trotz dieses Vorteils die Wellenlänge von 193 nm zur Mikrochirurgie am Auge nicht verwendet, da durch Streulicht bzw. durch nicht im Prozeß umgesetztes Wirklicht des Lasers die Gefahr mutagener Veränderungen gesunder Gewebsareale besteht. Neuere Untersuchungen haben gezeigt, daß die Bestrahlung der Hornhaut mit 193 nm-Strahlung neben der Photoablation die Emission kurzwelliger Fluoreszenz der Hornhaut sowie Strahlungsemission aufgrund des Photoablationsprozesses bewirkt. Bei Erhöhung der Laserstrahl-Energiedichte über die Wirkschwelle erhält der kurzwellige Anteil dieser Fluoreszenzstrahlung mehr Gewicht im Spektrum. Insbesondere tritt Strahlung auf, deren Wellenlänge auch im Bereich 290 bis 320 nm liegt. Da die Hornhaut oberhalb von 300 nm durchlässig ist werden diese Wellenlängen von ihr durchgelassen und von der Linse absorbiert. Strahlung in diesem Wellenlängenbereich hat eine eindeutig kataraktogene Potenz und ist deshalb besonders gefährlich für die Augenlinse.

Wird mit der Vorrichtung nach der Erfindung der Operationsstelle eine UV-absorbierende Substanz zugeführt, so werden intraokulare Strukturen vor den schädlichen Wellenlängen der Sekundärstrahlung abgeschirmt, die wie beschrieben bei der Photoablation der Hornhaut mittels 193 nm-Laserstrahlung entstehen.

Die Vorrichtung nach der Erfindung macht es möglich als Strahlquelle einen bei 193 nm emittierenden Laser einzusetzen um eine Photoablation der Hornhaut zu bewirken ohne die durch die entstehende Sekundärstrahlung verursachte Gefährdung intraokularer Strukturen in Kauf nehmen zu müssen.

Es hat sich gezeigt, daß die UV-absorbierenden Substanzen, die im Zusammenhang mit der 308 nm-Laserstrahlung Verwendung finden auch für die erforderliche Abschirmung bei der Verwendung von 193 nm-Laserstrahlung geeignet sind.

Als besonders vorteilhaft hat sich für die Mikrochirurgie am Auge die Verwendung eines bei 193 nm oder 308 nm strahlenden Excimer-Lasers in Verbindung mit einem im UV-Bereich absorbierenden Pharmazeutikum erwiesen, das ein Sulfonamid wie z.B. Sulfisomidin-Natrium oder Sulfacetamid als im UV absorbierenden Bestandteil enthält. Andere geeignete UV-absorbierende Substanzklassen sind: Sulfonamide, Tetrazykline, Lokalanasthetika wie z.B. Oxybuprocain-HCL oder Tetracain-HCL, Beta-Blocker wie z.B. Befunololhydrochlorid, Vitamine wie z.B. Vitamin B oder Vitamin C.

Weiterhin eignen sich Substanzen wie Indometacin, Azetazolamid, para-Amino-Benzoesäure, Pyridoxin-HCL, Pyrimethamine, Folsäure sowie ein unter der Bezeichnung "Evans blue" bekannter Stoff für den genannten Zweck. Alle diese Substanzen enthalten aromatische Ringsysteme, die für die UV-Absorption sorgen.

Die verwendete im Bereich der Wellenlänge der Laserstrahlung absorbierende Substanz sollte in einer Trägerflüssigkeit enthalten sein, deren Galenik die Penetration in das beaufschlagte organische Gewebe gewährleistet. Da ein gutes Penetrationsvermögen jedoch gleichzeitig dafür sorgt, daß die applizierte absorbierende Substanz auch wieder relativ schnell aus dem Bereich heraus diffundiert, in dem der chirurgische Eingriff stattfindet, ist es vorteilhaft, einen intermittierend betriebenen Laser zu verwenden und die absorbierende Substanz in der Zeit zwischen Laserimpulsen immer wieder aufs neue zuzugeben. Dies kann beispielsweise dadurch erfolgen, daß die absorbierende Substanz über eine Dosiereinrichtung zu der bei vielen Eingriffen ohnehin nötigen Spülflüssigkeit zugegeben wird.

In einem zweckmäßigem Ausführungsbeispiel der Erfindung ist zur Übertragung der Laserstrahlung auf das Gewebe eine Lichtleitfaser vorgesehen, deren Spitze eine derartig fokussierende Wirkung hat, daß die höchste Energiedichte etwa 1 mm vor der Faserspitze lokalisiert ist.

Die Erfindung wird im folgenden anhand verschiedener Ausführungsbeispiele, die sich auf die Mikrochirurgie am Auge beziehen näher erläutert. Die Figuren 1 - 18 der beigefügten Zeichnungen zeigen im einzelnen
- Figur 1: eine Prinzipskizze, die den Gesamtaufbau der erfindungsgemäßen Vorrichtung zeigt;
- Figur 2a 2b und 2c: vergrößerte Darstellungen verschiedener Ausführungsformen für die Stirnseite der verwendeten Glasfaser;
- Figur 3a: eine Schnittzeichnung eines für die Vitrektomie geeigneten Handstückes im Schnitt längs der Achse der darin gehaltenen Lichtleitfaser (3);
- Figur 3b und 3c: radiale Schnitte durch die Spitze (15) des in Figur 3a gezeigten Handstückes;
- Figur 4: eine Schnittzeichnung eines für die Linsenablation geeigneten Handstückes längs der Achse der darin geführten Lichtleitfaser;
- Figur 5: eine Schnittzeichnung eines für die Goniopunktur geeigneten Handstückes längs der Achse der darin geführten Lichtleitfaser;
- Figur 6a: eine Prinzipskizze die einen Versuchsaufbau zur Ermittlung der UV-Exposition der Retina während einer Linsenablation mit dem Handstück aus Figur 4 zeigt; einer Linsenablation mit dem Handstück aus Figur 4 zeigt;
- Figur 6b: eine Prinzipskizze die den Vorgang der Vitrektomie (Glaskörperchirurgie) mit dem Handstück aus Figur 3 zeigt;
- Figur 6c: eine Prinzipskizze, die den Vorgang der Gonioablation (fistulierende Glaukomoperation im Kammerwinkel) unter Verwendung des Handstücks aus Figur 5 zeigt;
- Figur 7: eine Prinzipskizze, die den Gesamtaufbau der erfindungsgemäßen Vorrichtung in Verbindung mit einem euzentrischen Mikromanipulator für die Hornhautchirurgie zeigt,
- Figur 8 und 9: Diagramme, in denen die Ablationsrate von Hornhäuten ohne bzw. mit Zugabe verschiedener absorbierender Substanzen dargestellt ist;
- Figur 10: ein Diagramm, das den Temperaturanstieg in der Hornhaut in Abhängigkeit von der Repetitionsrate des verwendeten Lasers ohne, bzw. bei Zugabe verschiedener UV-absorbierender Substanzen zeigt;
- Figur 11: ein Diagramm, in dem die Abhängigkeit der durch die Hornhaut transmittierten Strahlung bei 308 nm von der Anzahl der Tropfen verschiedener UV-absorbierender Substanzen dargestellt ist;
- Figur 12: Transmissionsspektren von 10 µm dicken Hornhautscheiben, die unbehandelt, bzw. mit 8 Tropfen verschiedene UV-absorbierender Substanzen behandelt wurden;
- Figur 13: ein Diagramm, in dem die Abhängigkeit der Breite der Nekrosezone einer mit UV-absorbierender Substanz vorbehandelten Hornhaut von der Anzahl der applizierten Tropfen dargestellt ist;
- Figur 14: ein Diagramm, in dem die Abhängigkeit der Schnittiefe durch die Hornhaut von der Repetitionsrate des verwendeten Lasers bei Zugabe verschiedener UV-absorbierender Substanzen dargestellt ist;
- Figur 15: ein Diagramm in dem die Breite der Nekrosezone einer mit UV-absorbierenden Substanz vorbehandelten Hornhaut von der Zeit zwischen Tropfenapplikation und Beginn der Laserbehandlung dargestellt ist;
- Figur 16: das Transmissionsspektrum der Hornhaut;
- Figur 17a: das Spektrum der bei Bestrahlung der Hornhaut mit 193 nm-Laserstrahlung unter der Wirkschwelle entstehenden und ins Augeninnere transmittierten Fluoreszenzstrahlung;
- Figur 17b: das Spektrum der bei Bestrahlung der Hornhaut mit 193 nm-Laserstrahlung oberhalb der Wirkschwelle entstehenden und ins Augeninnere transmittierten Fluoreszenzstrahlung;
- Figur 18: das Spektrum der bei Bestrahlung von mit einem UV-Absorber vorbehandelten Hornhaut mit 193 nm-Strahlung oberhalb der Wirkschwelle entstehenden und ins Augeninnere transmittierten Fluoreszenzstrahlung.

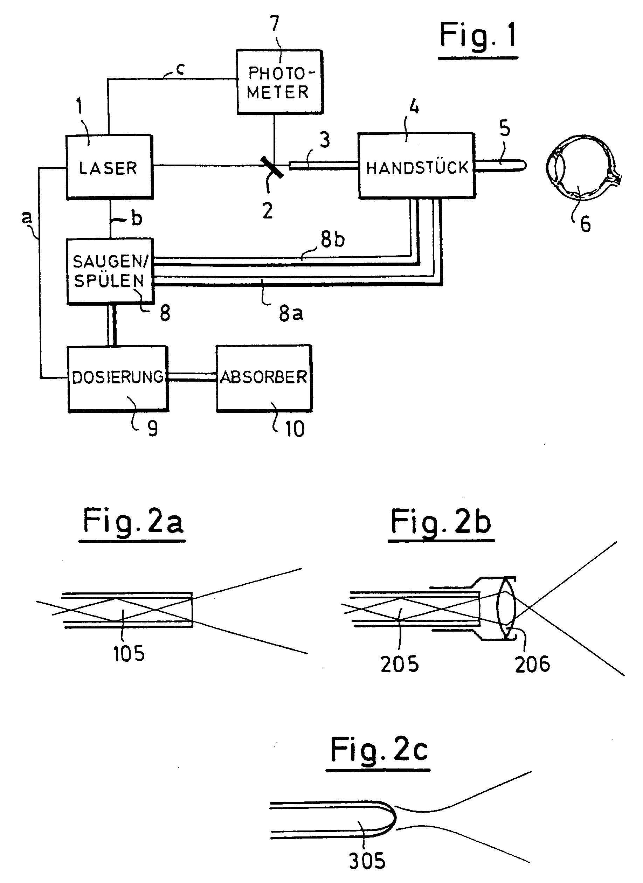

Die in der Prinzipskizze nach Figur 1 dargestellte Vorrichtung zur Chirurgie am Auge mittels Laserstrahlung enthält als Lasergenerator einen Xenonchlorid-Excimer-Laser (1), der bei einer Wellenlänge von 308 nm strahlt und Pulsbreiten zwischen 40 ns und 100 ns liefert. Der Strahl dieses Lasers (1) ist über einen teildurchlässigen Spiegel (2) geführt und auf das Ende einer Lichtleitfaser (3) aus Quarz fokussiert. Diese Lichtleitfaser (3) ist von einem Handstück (4) aufgenommen, in dessen Spitze (5) die dem Auge (6) zugewandte Stirnseite der Faser (3) mündet.

Wie nachher noch beschrieben wird, sind verschiedene Handstücke (4/5) auswechselbar vorgesehen, um die unterschiedlichen chirurgischen Aufgabenstellungen am Auge durchführen zu können.

In dem über den Teilerspiegel (2) ausgespiegelten Teilstrahlengang ist ein Photometer (7) angeordnet. Dieses Photometer (7) mißt vom Auge rückgestreute Strahlung, beispielsweise Streustrahlung auf der Laserwellenlänge oder Fluoreszenzstrahlung. Das Photometer (7) ist über einen Steuerausgang c mit dem Lasergenerator (1) verbunden so daß dann, wenn die rückgestreute Strahlung vorbestimmte Grenzwerte über- oder unterschreitet, der Laser (1) abgeschaltet werden kann.

Das Handstück (4) ist über ein System von Saugleitungen (8a) bzw. Spülleitungen (8b) mit einem geregelten Saug/Spülgerät (8) im Lasergrundgerät verbunden. An die Saug/Spüleinheit (8) ist weiterhin eine Dosiereinheit (9) angeschlossen, von der gezielt Mengen einer in einem Vorratsgefäß (10) enthaltenen UV-absorbierenden Substanz der Spülflüssigkeit intermittierend zugegeben werden können. Die Dosiereinheit ist außerdem ebenso wie die Saug/Spüleinheit (8) über Steuerleitungen a bzw. b mit dem Lasergenerator (1) so verbunden, daß der Laser (10) während der Zugabe der absorbierten Substanz abgeschaltet und nach einer vorbestimmten Pausenzeit wieder eingeschaltet wird. In einer weiteren Ausbaustufe ist es möglich, daß der Laser, die Saug/Spüleinrichtung (8) sowie die Dosiereinrichtung (9) von einem Rechner bzw. Mikroprozessor gesteuert wird.

Die Arbeitsweise der vorstehend beschriebenen Vorrichtung bzw. die mit ihr durchgeführten Verfahren für die Indikationen Glaukomoperation, Vitrektomie, Linsenkernentfernung und refaktive Hornhautchirurgie werden nachstehend beispielhaft beschrieben:

### Beispiel 1

### Fistulierende Glaukomoperation im Kammerwinkel

Mit diesem Operations-Verfahren wird ein Kanal zwischen der Vorderkammer und dem Subkonjunktivalraum im Auge geschaffen. Hierfür hat sich auch der Ausdruck "Sklerostomie" eingebürgert. Synonym hierzu wird dann, wenn für diesen Eingriff ein Excimer-Laser verwendet wird, auch der Name "Gonioablation" gebraucht.

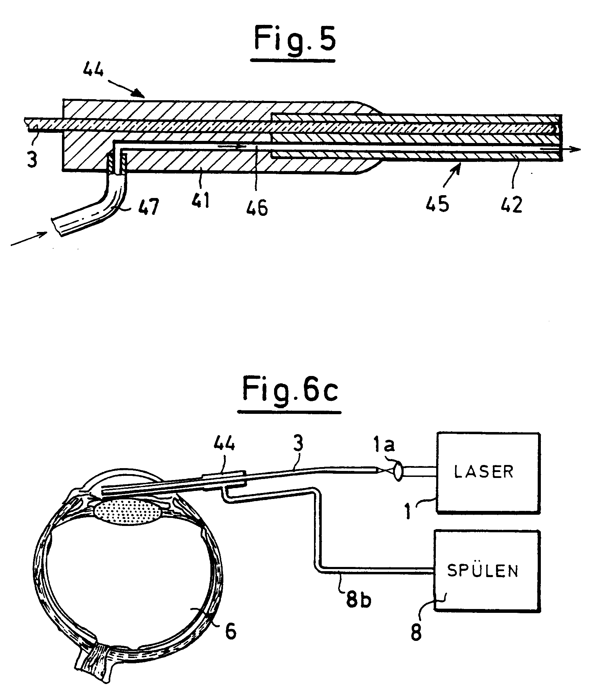

Das für diesen Eingriff verwendete Handstück ist in Figur 5 dargestellt. Es besteht aus einem Handgriff (44), der an seinem vorderen Ende in eine etwa 1 mm starke Metallfassung (45) übergeht. In diese Metallfassung (45) sind die Quarzfaser (3) und ein Spülkanal (46) eingebettet. Der Spülkanal (46) mündet in einen Anschlußstutzen (47) im Handgriff und tritt am anderen Ende stirnseitig aus der Fassung (45) aus.

Die Stirnseite (305) der Faser (3) besitzt fokussierende Eigenschaften und ist deshalb wie in Figur 2c dargestellt konvex gerundet. Dadurch ist gewährleistet, daß die höchste Energiedichte etwa 1 mm vor der Faserspitze lokalisiert ist, was ein berührungsfreies Schneiden ermöglicht. Im Gegensatz hierzu ist die höchste Energiedichte bei Fasern mit ebener Stirnseite (105), wie sie bislang für diesen Zweck verwendet wurden, (Figur 2a) direkt an der Oberfläche der Faser konzentriert, so daß kein wirklich berührungsfreies Schneiden möglich ist und die Gefahr einer vorzeitigen Zerstörung der Faserspitze besteht. Zwar ist auch schon vorgeschlagen worden, dieses Problem zu umgehen, in dem man wie in Figur 2b dargestellt vor der Faser (205) eine mit einer Fassung versehene Konvexlinse (206) anbringt. Damit wird jedoch der Durchmesser der Faserspitze vergrößert, was in vielen Fällen hinderlich ist und das Einführen in Handstücke erschwert.

In Versuchen an isolierten Schweine- und Schafsaugen konnte mittels eines über eine Quarzfaser in die Vorderkammer eingekoppelten Excimer-Laser-Strahls bei 308 nm im Bereich des Trabekelwerks eine Fistel zwischen der Vorderkammer und dem Raum unter der Bindehaut geschaffen werden, ohne daß dabei die Bindehaut verletzt wurde. Um die Vorderkammer während des intraokularen Eingriffs gestellt zu halten, wurden die Vorversuche mit einer Vorderkammerinfusion durchgeführt, bei der der Zugang am Limbus erfolgte. Der bei der Gonioablation in der Vorderkammer herrschende Druck wurde hierbei durch die Höhe der Infusionsflasche variiert. Die Durchführung der Gonioablation mit dem Handstück nach Figur 5 ist in der Prinzipskizze nach Figur 6c dargestellt. Nach der Öffnung des Auges am Limbus mit einer Diszissionsnadel wird die Spitze (45) des Handstücks (44) in die Vorderkammer eingeführt und in den gegenüberliegenden Kammerwinkel des Auges (6) vorgeschoben. Während gleichzeitig minimaler Druck auf das Handstück ausgeübt wird, erfolgt das Einschalten des Lasers (1), der über die Optik (1a) auf das hintere Ende der Quarzfaser (3) fokussiert ist. Durch die Einwirkung der Laserstrahlung entstehen Gasbläschen im Kammerwinkel. Der Moment der Perforation im Bereich des corneoskleralen überganges ist bei geeigneter Handhabung spürbar. Nach Zurückziehen des Handstückes bildet sich spontan ein Sickerkissen aus, die bei der Gonioablation entstandenen Gasbläschen sind dann in die Vorderkammer aufgestiegen.

Das Kollabieren der Vorderkammer wird durch den Spülkanal im Handstück (44) verhindert, der über die Spülleitung (8b) an die Saug/Spüleinheit (8) angeschlossen ist.

Die vorstehend beschriebene Sklerostomie wurde nicht nur mit einem Excimer-Laser sondern für vergleichende Untersuchungen auch mit einem Argon-Laser bei 488 nm und 514 nm sowie mit einem Dauerstrich Nd-YAG-Laser bei 1064 nm durchgeführt. Die histologische Untersuchung der operierten Augen zeigte, daß sich für den Nd-YAG-Laser (applizierte Leistung 20-40 Watt bei 0,1 Sekunden Pulsdauer) sowie für den Argon-Laser (angewandte Leistung 0,3-3 Watt bei Pulsdauern zwischen 0,02 und 1 Sekunde) Nekrosebreiten zwischen 300 µm und 700 µm ergaben. Im Gegensatz dazu zeigen die mit dem Excimer-Laser bei der Wellenlänge 308 nm erzeugten Fistelkanäle (Pulsenergie zwischen 2 mJ und 10 mJ bei einer Repetitionsrate von 20 Hz) nur eine 40-60 µm breite Nekrosezone.

Die Breite dieser Nekrosezone konnte auf ein Fünftel reduziert werden, wenn der Spülflüssigkeit ein UV-absorbierendes Medikament vor dem Einschalten des Lasers zugegeben wurde. Gleichzeitig reduzierte sich die Zahl der benötigten Laserpulse und damit die applizierte Gesamtenergie um die Hälfte. Ein solches Medikament ist beispielsweise Sulfisomidin-Natrium 1 H₂O 114,4 mg/ml entsprechend 100 mg/ml Sulfisomidin. Dieses nachfolgend "Substanz A" genannte Medikament wird in Europa unter dem Handelsnamen "Aristamid" Augentropfen angeboten.

Ein anderes, geeignete UV-absorbierende Substanzen enthaltendes Medikament sind Augentropfen, die unter der Bezeichnung "Blephamide Liquifilm" angeboten werden. Dieses nachfolgend als "Substanz B" bezeichnete Medikament setzt sich zusammen /ml aus Sulfacetamid-Natrium 105 mg, Prednisolon-21-acetat 2,20 mg, Phenylephrinhydroclorid 1,2 mg, Phenanzon 1,0 mg und Polyvinylalkohol als Trägersubstanz 14,0 mg. Wirksamer UV-absorbierender Bestandteil ist hierin Sulfacetamid.

### Beispiel 2

### Vitreoablation: Vitrektomie mit dem Excimer-Laser über eine Glasfaser

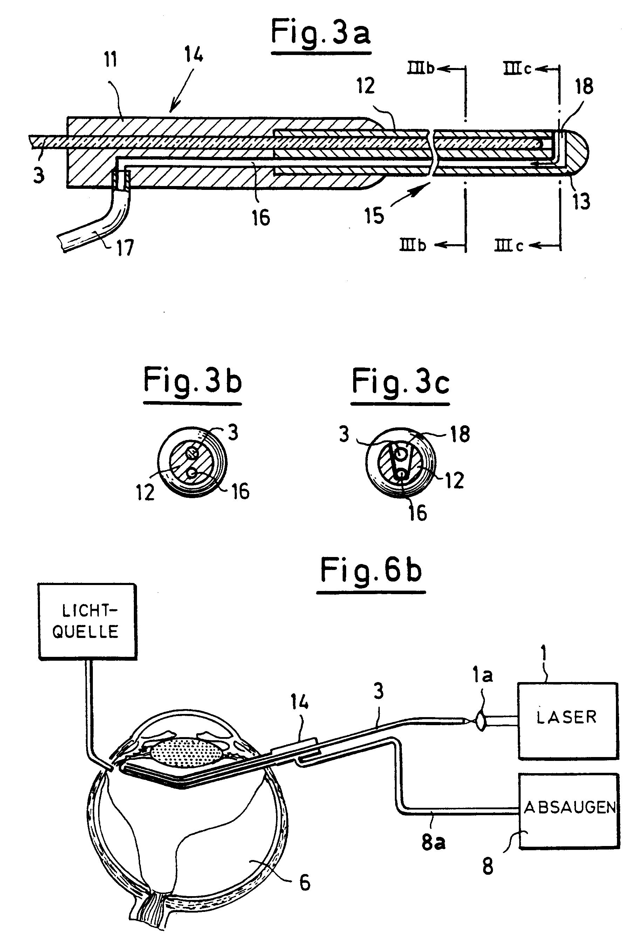

Für die Ausführung dieses Eingriffes wurde das in Figur 3a-c dargestellte Handstück verwendet. Es ist ähnlich aufgebaut wie das Handstück für die Gonioablation in Figur 5 und besitzt einen Griff (14), der am vorderen Ende in ein dünnes, ca. 1 mm starkes Metallrohr (12) übergeht. In einem der zwei übereinanderliegenden Kanäle im Handstück (14) ist die Quarzglasfaser (3) gefaßt, während der andere Kanal (16) als Saugkanal ausgebildet ist und in einen Sauganschluß (17) mündet. Die Faser (3) ist im Handstück so befestigt, daß ihre Spitze gegenüber der Mündung des Saugkanals (16) etwas zurückspringt, um einen Kontakt abgesaugten Gewebes mit der Faseroberfläche zu vermeiden. Außerdem trägt die Metallfassung (12) der Spitze (15) des Handstücks stirnseitig eine abschirmende Blende (13). Diese Blende (13) dient dazu, die Retina gegen achsial aus der Faser austretende Laserstrahlung abzuschirmen.

Bei der Vitrektomie wird der Glaskörper durch die seitliche Öffnung (18) zwischen der Blende (13) und der Stirnseite der Faser (3) angesaugt und von dem etwa im Saugkanal liegenden Laserfokus geschnitten. Durch den Saugkanal (16) wird das geschnittene Glaskörpergewebe abgesaugt. Die Ansaugöffnung für den Glaskörper muß kleiner als der Strahl des aus der Faser (3) austretenden Excimer-Lasers sein, damit nur geschnittenes Glaskörpergewebe angesaugt wird, da andernfalls eine Verstopfung des Saugkanals resultiert.

Gleichzeitig dient der Saugkanal (16) bzw. der bei einer Vitrektomie ohnehin vorhandene Infusionszugang zur intermittierenden Zugabe von UV-absorbierenden Substanzen, die die Ablationsschwelle des Glaskörpergewebes absenken und das übrige Augengewebe, insbesondere die Retina vor UV-Streustrahlung abschirmen.

In ersten Vorversuchen konnte gezeigt werden, daß sich Glaskörpergewebe mit dem Excimer-Laser bei 308 nm und dem in Figur 3 dargestellten Handstück schneiden läßt. Hierbei wurden auch Messungen der Ablationsraten für Glaskörpergewebe durchgeführt. Ein isolierter Glaskörper wurde in einer Küvette ablatiert und abgesaugt. Aus der Differenz des Gewichts der Proben vor und nach der Ablation wurde die Ablationsrate ermittelt. Als Schwelle für die Glaskörperablation gab sich dabei etwa ein Wert von 5 mJ/mm². Die bei einer Repetitionsrate (Pulsfrequenz des Lasers) von 20 Hz ermittelten Ablationsraten liegen für eine 600 µm starke Faser, wie sie im Handstück nach Figur 3 verwendet wurde, im Bereich von 200 mg/min bei einer Pulsenergie von 15 mJ.

Wurde dem Glaskörper vorher eine UV-absorbierende Substanz zugeführt, ergab sich eine drastische Erhöhung der Ablationsrate auf Werte über 1 g/min. Durch diese Erhöhung der UV-Absorbtion des Glaskörpers ist auch eine effektive Abschirmung der Retina während der Vitreoablation gegeben.

Wird die Vitreoablation intraoperativ durchgeführt, wie dies in der Prinzipskizze nach Figur 6b dargestellt ist, dann arbeitet man zweckmäßig mit einem Handstück, das wie dort dargestellt eine abgewinkelte bzw. gekrümmte Spitze besitzt. Dies erlaubt eine Entfernung des Glaskörpers an der Glaskörperbasis, ohne durch Berühren der hinteren Linsenoberfläche eine Kataraktbildung zu induzieren.

Die Zugabe des UV-absorbierenden Medikamentes erfolgt dann intermittierend zum Saug/Schneidevorgang durch den Saugkanal (16) des Vitrektomiehandstücks (14) hindurch. Dies läßt sich durch eine entsprechende Steuerung der Saug/Spüleinheit (8) erreichen.

### Beispiel 3

### Endokapsuläre Phakoablation mit einem Excimer-Laser über eine Quarzfaser

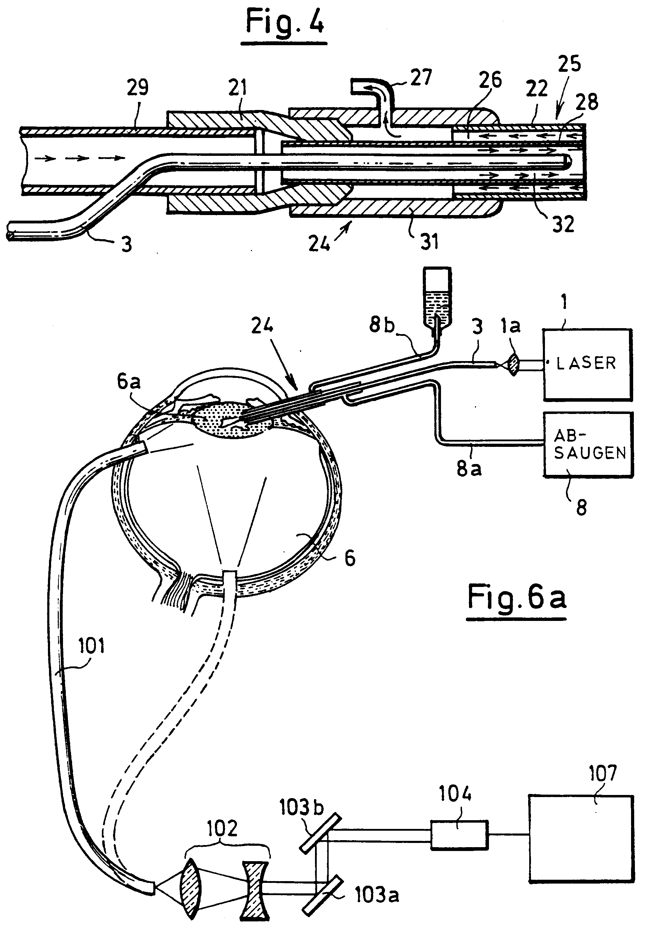

Das Handstück (24) für diesen Eingriff ist in Figur 4 dargestellt. Es besteht aus einem zylindrischen Handgriff (31), an den ein Saugstutzen (27) angesetzt ist. In das hintere Ende des Zylinders (31) ist ein Kanülenschaft (21) eingesetzt, in den ein Spülschlauch (29) mündet. Das vordere Ende des Kanülenschaftes (21) hält innen ein Kanülenrohr (28), das den inneren Spülkanal (32) von einem äußeren Saugkanal (26) trennt. Der Saugkanal (26) wird durch ein zweites, koaxial um das innere Kanülenrohr (28) herum gelegtes Kanülenrohr (22) gebildet, das in das vordere Ende des Handgriffes (31) eingesetzt ist. Im Inneren des Spülkanals ist außerdem die Glasfaser (3) gehalten, deren vorderes, gerundetes fokussierendes Ende ca. 1 mm hinter die Spitze des Handstücks (24) zurückspringt. Die Faser wird so ständig mit einer Lösung umspült, um die Ablationsprodukte von der Faserspitze fernzuhalten. Das Absaugen der Linsenfragmente erfolgt durch den Saugkanal (26) hindurch, der konzentrisch um den Spülkanal (32) geführt ist.

Mit diesem Handstück gelang die endokapsuläre Phakoablation bei nur minimalem Schaden an der Linsenkapsel. Zur Verdeutlichtung des Ablaufes wird auf die Darstellung nach Figur 6a verwiesen. Die Öffnung in der Linsenkapsel (6a) wurde nach einer in der Literatur beschriebenen Methode mit einem Thermokauter gesetzt. Das Handstück (24) mit der darin integrierten Quarzglasfaser wurde durch diese Öffnung in die Linse (6a) vorgeschoben. Zunächst wurde dann die vor dem Kern liegende Rinde ablatiert und abgesaugt. Danach erfolgte die Ablation des Kerns. Nach vollständiger Ablation und Absaugen des Kerns wurde die Rinde konventionell abgesaugt. Zur Kontrolle des Ergebnisses wurde der leere Kapselsack mit einem optisch transparentem Gel (Methylcellulose) gefüllt.

Um abzuklären, wie stark die Retina während der Phakoablation mit UV-Strahlung belastet wird, wurde in einem Versuch mit einem isolierten Rinderauge die UV-Strahlung, die die Netzhaut erreicht, intraoperativ gemessen. Im Maculabereich bzw. im Bereich der peripheren Retina wurde eine 1 mm starke Quarzglasfaser (101) plaziert und die davon aufgefangene UV-Strahlung durch ein Galilei-Teleskop (102) gebündelt und über zwei dielektrische Spiegel (103a) und (103b), die nur die Wellenlänge 308 nm des Lasers (1) reflektierten, auf eine Photodiode (104) abgebildet. Die Photodiode (104) war mit einem kalibrierten Energiemeßgerät (107) verbunden. Durch diese Anordnung war gewährleistet, daß das Licht des Operationsmikroskops sowie die bei der Ablation der Linse entstehende Eigenfluoreszenz der Linse nicht mit gemessen wurde.

Sowohl im Bereich der Macula wie auch in der peripheren Retina war die während der Phakoablation gemessene Gesamtenergiedichte deutlich kleiner als die Schwelle für einen Retinaschaden, der für die Wellenlänge 308 nm bei 5 J/cm² liegt. Eine weitere drastische Senkung der Energiedichte auf der Retina wurde durch intermittierende Zugabe der eingangs genannten UV-absorbierenden Medikamente (Substanz A bzw. Substanz B) in die Spülflüssigkeit erreicht. Experimente an Schlachthofaugen haben gezeigt, daß sogar durch eine Tropfenapplikation der UV-absorbierenden Medikamente auf der Hornhaut eine ausreichende Konzentration im Glaskörper des Auges (6) erreicht wird, um die Retina effektiv vor der Streustrahlung des Lasers bei der Wellenlänge 308 nm abzuschirmen.

### Beispiel 4

### Refraktive Hornhautchirurgie mit dem Excimer-Laser bei 308 nm über Glasfaser

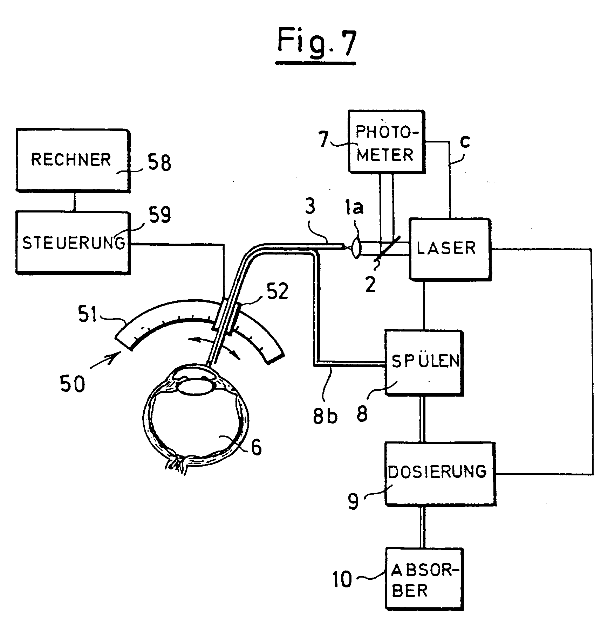

Für diesen Eingriff, der durch die Prinzipdarstellung nach Figur 7 verdeutlicht wird, ist zusätzlich die Anwendung eines euzentrischen Mikromanipulators vorgesehen, mit dem nach Einprogrammierung der Raumkurve für die Schnittführung exakt die Geometrie der Cornea in diversen Schnittrichtungen nachgeführt und reproduziert werden kann. Solche Mikromanipulatoren sind an sich bekannt. Sie bestehen aus einer bogenförmigen Führung (51), auf der ein Träger (52) geführt und elektrisch angetrieben positioniert werden kann. Für die Positionierung sind ein Rechner (58) sowie eine diesem nachgeschaltete Steuereinheit (59) vorgesehen. Im Halter (52) sind die Quarzglasfaser (3) und eine Spülrohr, beispielsweise die Spitze (45) des in Figur 5 dargestellten Handstückes (44) befestigt. Auf diese Weise kann der Strahl des in die Faser (3) über die Optik (1a) eingekoppelten Excimer-Lasers (1) konzentrisch zur Hornhautkrümmung über diese hinweggeführt werden. Die Saug/Spüleinrichtung (8) entspricht der nach Figur 1 und ist wieder über eine Dosiereinheit (9) mit einem Vorratsgefäßt (10) verbunden, in dem sich eines der vorstehend genannten Medikamente (Substanz A bzw. Substanz B) befindet. Die Spülleitung (8b) dient neben der Medikamentenzugabe zur intraoperativen Befeuchtung der Hornhaut mit physiologischer Kochsalzlösung. Weiterhin ist ein Photometer (7) vorgesehen, das die von der Hornhaut rückgestreute und von der Faser (3) zurückübertragene Fluoreszenzstrahlung nach Auskopplung über den Teilerspiegel (2) mißt und über die Steuerleitung c den Lasergenerator (1) abschaltet, wenn ein zu hohes Fluoreszenzsignal eine ungenügende Konzentration des UV-absorbierenden Medikamentes in der Hornhaut anzeigt.

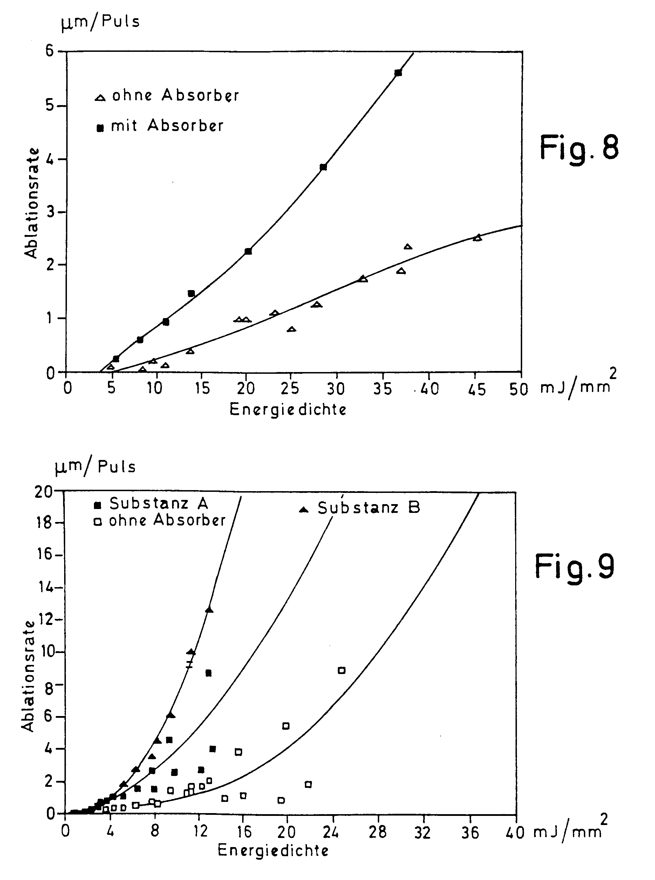

Zur Ermittlung der Ablationsraten wurden Hornhäute von Schweinen bzw. Schafen bis zur Perforation bestrahlt und die dazu nötigen Laserpulse gezählt. Messungen der Dicke des Hornhautzentrums mit einer elektronischen Schublehre sowie mit einem Mikroskop mit Okularskala ergaben gut reproduzierbar eine Hornhautdicke von 0,7 mm, die für die Untersuchung zugrundegelegt wurde. Die Ablationsrate wurde aus der Zahl der zur Perforation einer Hornhaut nötigen Laserpulse ermittelt. Bei der Messung der Ablationsraten unter Verwendung von geeigneten UV-absorbierenden Substanzen ließ sich nachweisen, daß alle einleitend genannten Absorber die Ablationsschwelle herabsetzen und die Ablationsrate deutlich steigerten. Hierzu wird auf die Darstellung nach Figur 8 und Figur 9 verwiesen. Das Diagramm nach Figur 8 wurde an einer Schweinehornhaut bei der Wellenlänge 308 nm ermittelt, während die Figur 9 die Ablationskurven von Schafhornhäuten mit und ohne UV-Absorber wiedergibt. In beiden Fällen ist die Ablationsrate in Mikrometern pro Puls gegen die Energiedichte der Laserstrahlung vor der Faserspitze in mJ pro Quadratmillimeter aufgetragen. Man erkennt, daß die Zugabe der eingangs genannten Substanzen A und B die Ablationsrate drastisch, teilweise mehr als einen Faktor 2 erhöht und außerdem die Ablationsschwelle, d.h. die Energiedichte, ab der überhaupt erst Ablation eintritt, senkt. Die Zugabe der absorbierenden Substanz erlaubt es daher, mit geringeren Energiedichten zu arbeiten und dennoch den Eingriff zu verkürzen, wodurch die UV-Exposition des Augengewebes deutlich verringert wird.

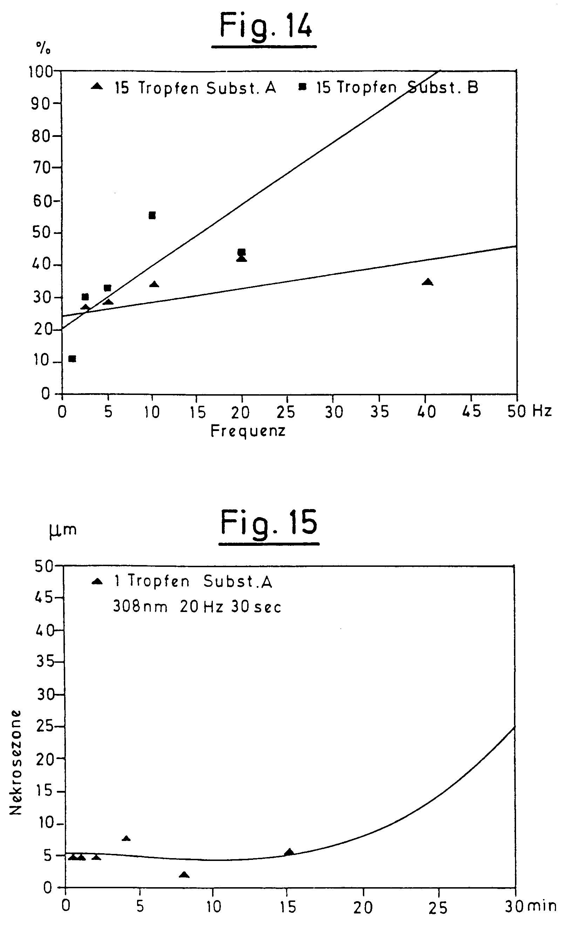

Die Schnittiefe pro Puls ist eine Funktion der verwendeten Pulsrate. Dieser Zusammenhang ist in Figur 14 dargestellt. Dort ist die von 100 Laserpulsen bewirkte Schnittiefe in der Hornhaut in Prozent der Hornhautdicke gegen die Repetitionsfrequenz des Lasers in Hz aufgetragen. Aus der Darstellung ist die höhere Effektivität der Substanz B zu erkennen, die mit 100 Laserpulsen bei einer Repetitionsrate von 40 Hz Schnitte durch die gesamte Hornhautdicke ermöglicht.

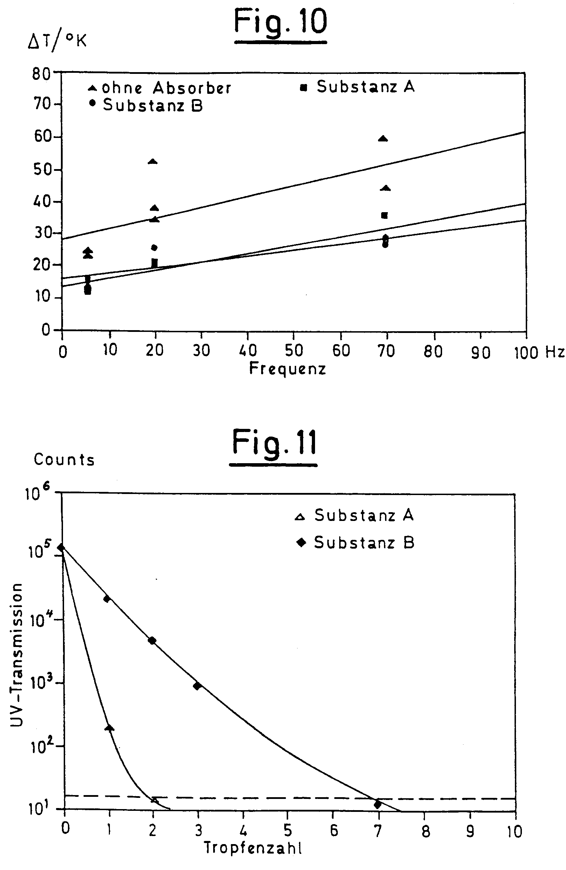

Gleichzeitig senken sowohl die Substanz A wie auch die Substanz B den während des Schnittes stattfindenden Temperaturanstieg in der Hornhaut. Dies ergibt sich klar aus dem Diagramm nach Figur 10, in dem der Temperaturanstieg in Grad gegen die Repetitionsrate des Lasers in Hz für Hornhäute von Schafen unbehandelt bzw. nach Zugabe der Substanz B und der Substanz A jeweils in Abständen von 5 Minuten über den Zeitraum einer Stunde hinweg aufgetragen ist.

Außerdem wird durch die applizierten UV-Absorber (z.B. Substanz A, Substanz B) die Augenlinse effektiv vor der Strahlung des Lasers bei der Wellenlänge von 308 nm abgeschirmt. Diese Ergebnisse wurden auch in einer quantitativen Messung bestätigt. Dabei wurde eine 1000 µm dicke Faser vom hinteren Pol des Auges durch den Glaskörper und die Linse von Schafsaugen hindurch in die Vorderkammer vorgeschoben. In Figur 11 ist die von der Faser gemessene UV-Transmission der mit den Substanzen A und B behandelten Hornhaut in Abhängigkeit von der Tropfenzahl des Medikamentes aufgetragen. Aus der Darstellung läßt sich ablesen, daß wenige Tropfen bereits ausreichen, um das durch die Hornhaut transmittierte UV-Licht unter die Nachweisgrenze zu senken.

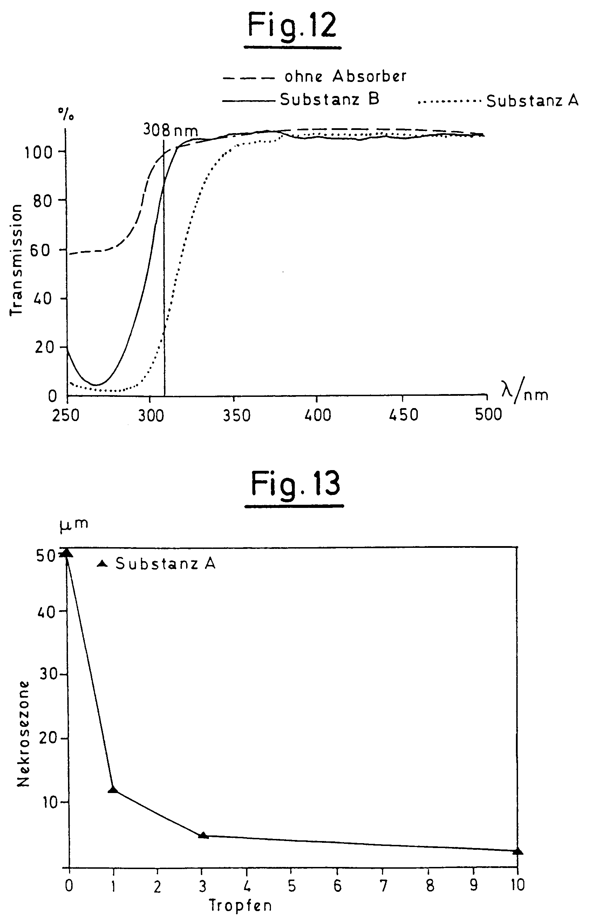

Dieses Ergebnis läßt sich gut mit den entsprechenden Absorptionsspektren von 10 µm dicken Hornhautscheiben korrelieren, die mit den entsprechenden Medikamenten behandelt waren. Diese Spektren sind in Figur 12 dargestellt. Man erkennt, daß im Wellenlängenbereich unter etwa 340 nm die Transmission der mit Substanz A bzw. Substanz B behandelten Hornhäute deutlich geringer ist als die Transmission von unbehandelten Hornhäuten.

Außerdem konnte eine durch die UV-Absorber bewirkte Verbesserung der Schnittqualität anhand von histologischen Untersuchungen nachgewiesen werden. Histologische Schnitte eines mit 1 J/cm² durchgeführten Laserschnittes in einer nativen Hornhaut ergeben etwa 200 µm breite Nekrosezonen direkt an den Schnitträndern, in denen man auch Vakuolen findet. An diese Nekrosezone schließt sich eine etwa 500 µm breite Zone mit einer Strukturauflockerung an. Bei kleinerer Energiedichte unterhalb der Schwelle resultiert kein Schnitt, sondern nur eine ähnliche Nekrosezone mit Vakuolen. Bestrahlt man bei der gleichen Energiedichte ein mit der Substanz A behandeltes Auge, dann ergibt sich auch unterhalb der Schwellenenergiedichte für die native Hornhaut wieder ein Schnitt, die Nekrosezone an den Schnitträndern beträgt nun nur noch etwa 2 bis 5 µm.

Die Abhängigkeit der Nekrosebreite von der Anzahl der Tropfen des verwendeten Medikamentes zeigt Figur 13. Bereits mit wenigen Tropfen der Substanz A läßt sich die Breite der Nekrosezone um mehr als eine Größenordnung senken. Eine Steigerung der Tropfenzahl über drei Tropfen hinaus hingegen bewirkt keine drastische Änderung mehr.

Die Zeit zwischen der Tropfenapplikation und dem Einschalten des Lasers darf nicht zu lange sein, da die Hornhautkonzentration des UV-Absorbers durch Diffusion ins Augeninnere nach längeren Wartezeiten hin abnimmt. Dies läßt sich vorteilhaft dadurch vermeiden, daß das Medikament in die in den Schneideapplikator (Handstück) integrierte Spülleitung eingegeben wird, so daß das Medikament bei Bedarf nachdosiert werden kann. Dies erfordert einen intermittierenden Betrieb der Vorrichtung.

Die maximale Wartezeit zwischen Medikamentenzugabe und dem Beginn des Laserschnittes kann experimentell bestimmt werden. Hierzu wurden verschiedene Schnitte in unterschiedlichen Zeitabständen nach Medikamentenzugabe durchgeführt und die Breite der Nekrosezone nach jedem Schnitt bestimmt. Das Ergebnis zeigt die Darstellung nach Figur 15. Bei Wartezeiten über 15 Minuten steigt die Breite der Nekrosezone wieder an, da der UV-Absorber (Substanz A) aus der Hornhaut heraus ins Augeninnere wegdiffundiert.

Die anhand des Beispiels 4 erläuterte Hornhautchirurgie läßt sich auch mit einem, bei 193 nm emittierenden Excimer-Laser, beispielsweise einem Argon-Fluorid-Laser durchführen. Da diese Wellenllänge zum derzeitigen Zeitpunkt nicht durch eine Lichtleitfaser geführt werden kann, ist die in Fig. 7 verwendete Faser (3) durch ein optisches System bekannter Art zu ersetzen, welches die von der Laserquelle ausgehende Strahlung auf die Hornhaut des Auges (6) fokussiert. Bei einer Bewegung des fokussierten Laserflecks über die Hornhaut kann der Laser mitbewegt werden oder es kann eine Spiegelanordnung vorgesehen sein, die das Laserlicht dem bewegten optischen System nachführt.

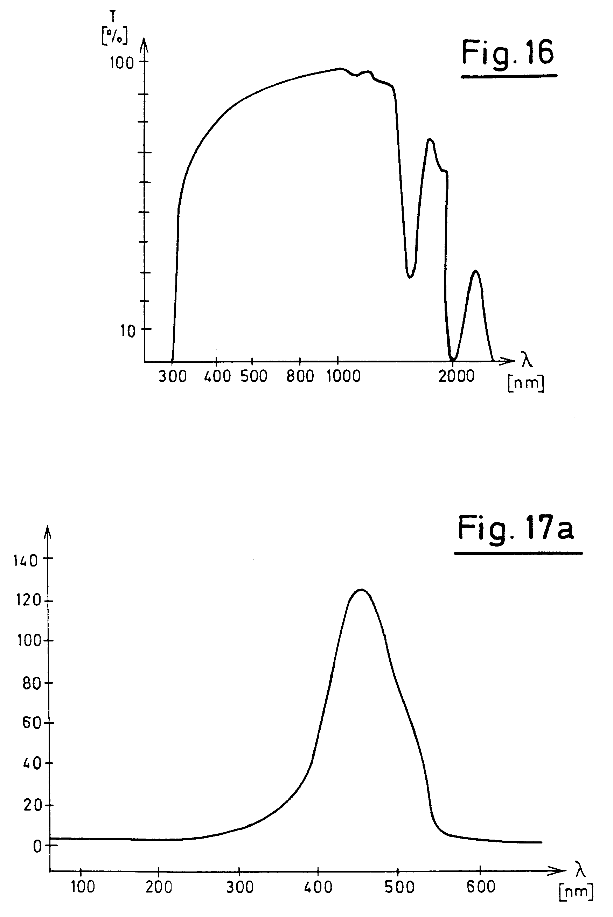

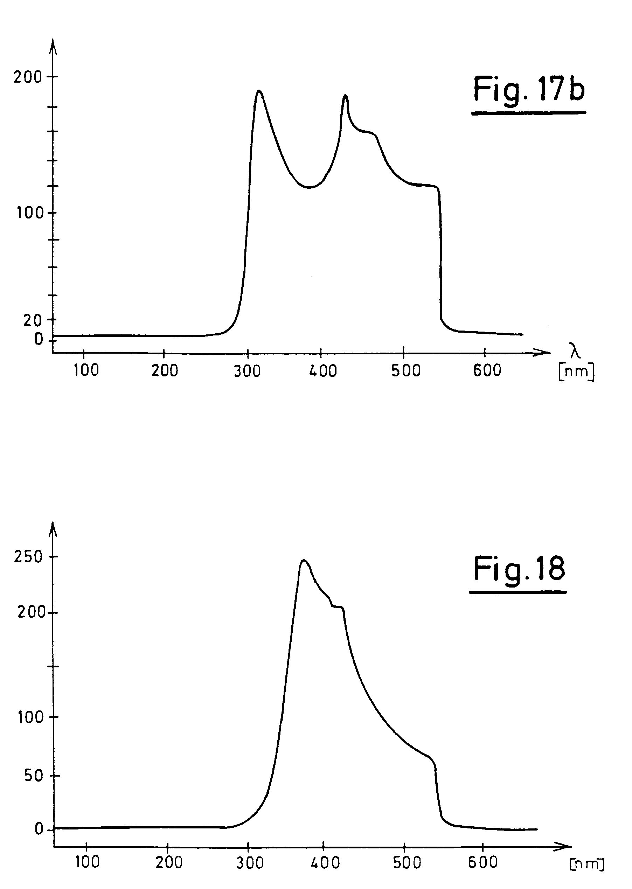

Wie Fig. 17a zeigt wird bei einer Bestrahlung der Hornhaut mit 193 nm-Laserstrahlung eine Fluoreszenzstrahlung ausgelöst, die entsprechend der Transmissionskurve der Fig. 16 in's Augeninnere gelangt. Wird im Gegensatz zu Fig. 17a die Energiedichte der Laserstrahlung über die Wirkschwelle hinaus erhöht, so daß Photoablation bewirkt wird, so erhält der kurzwellige Anteil der Sekundärstrahlung mehr Gewicht im Spektrum, wie Fig. 17b zeigt. Da die Hornhaut oberhalb von 300 nm transparent ist, wird diese kurzwellige Strahlung durchgelassen und von der Linse absorbiert. Untersuchungen haben gezeigt, daß Wellenlängen zwischen 290 und 320 nm aufgrund ihrer kataraktogenen Potenz besonders gefährlich für die Linse sind. Die Schwelle zur Erzeugung von Linsentrübungen bei diesen Wellenlängen liegt bei 600 mJ/cm².

Fig. 18 zeigt die Verhältnisse, wenn der vom Laserstrahl beaufschlagten Hornhaut eine UV-absorbierende Substanz z.B. Oxybuprocain zugeführt ist. Dies kann beispielsweise über die Spülleitung 8b der Fig. 7 geschehen.

Man erkennt aus Fig. 18, daß die schädliche Sekundärstrahlung zwischen 290 und 320 nm nahezu vollständig absorbiert wird und demzufolge im Spektrum nicht mehr auftritt.

Damit läßt sich die Laserstrahlung der Wellenlänge von 193 nm für die Hornhautchirurgie verwenden, ohne daß intraokulare Strukturen durch schädliche Strahlung gefährdet werden. Dies bringt neben dem Vorteil einer vergrößerten Ablationsrate gegenüber der 308 nm-Laserstrahlung den bedeutenden Vorteil mit sich, daß sich die thermischen Nekrosezonen um den eigentlich abzutragenden Bereich herum um den Faktor 20 - 50 verkleinern lassen. Es wird also eine optimale Schnittpräzision erreicht, wobei intraokulare Strukuren vor den schädlichen Wellenlängen der Sekundärstrahlung, die bei der Photoablation der Hornhaut mittels 193 nm-Laserstrahlung entstehen hinreichend abgeschirmt sind.

Die Erfindung ist vorstehend anhand der Mikrochirurgie am Auge erläutert worden. Dies bedeutet keine Einschränkung bezüglich der Anwendung der Vorrichtung nach der Erfindung. Diese Anwendung liegt ganz allgemein bei der medizinischen Chirurgie von biologischem Gewebe, bei der neben UV-emittierenden Excimer-Lasern auch Laser verwendet werden, die in anderen Wellenlängenbereichen emittieren.

## Patentansprüche

1. Vorrichtung zur Chirurgie von biologischem Gewebe mit
- einem intermittierend betriebenen Lasergenerator (1),
- Mitteln (3) zum Übertragen der Laserstrahlung zum Operationsfeld, die von einem Handstück (4, 14, 24, 44) so aufgenommen werden, daß deren Stirnseite auf das Operationsfeld gerichtet ist,
- sowie einer zusätzlichen Dosiereinheit (9) für die Zufuhr einer im Bereich der Wellenlänge des Laserstrahls absorbierenden Substanz zum Operationsfeld,
dadurch gekennzeichnet, daß die Vorrichtung weiter enthält
- eine Saug- und Spüleinrichtung (8) zum Entfernen des von der Laserstrahlung abgetragenen Gewebes mit dem Handstück (4, 14, 24, 44) verbunden ist und
- eine Elektronikeinheit vorgesehen ist, welche die Zufuhr der absorbierenden Substanz durch die Dosiereinheit (9) in der Zeit zwischen den Laserpulsen steuert.

2. Vorrichtung nach Anspruch 1, dadurch gekennzeichnet, daß als Mittel zur Übertragung der Laserstrahlung eine Lichtleitfaser (3) vorgesehen ist, die vom Handstück (4) aufgenommen wird, in dessen Spitze (5) die dem Operationsfeld zugewandte Stirnseite der Lichtleitfaser (3) mündet.

3. Vorrichtung nach Anspruch 2, dadurch gekennzeichnet, daß eine zur Übertragung der Laserstrahlung dienende Lichtleitfaser (3) und der Spülkanal (46) der Saug- und Spüleinrichtung (8) in den Handgriff (44) des Handstücks (4) eingebettet sind und an dessen Stirnseite enden, die auf das Operationsfeld gerichtet ist.

4. Vorrichtung nach Anspruch 2 oder 3, dadurch gekennzeichnet, daß die dem Operationsfeld zugewandte Stirnseite der Lichtleitfaser (3) konvex gerundet ist, so daß sie fokussierende Eigenschaften aufweist.

5. Vorrichtung nach Anspruch 1, dadurch gekennzeichnet, daß ferner eine Detektoreinheit (7) vorgesehen ist zum Messen von Strahlung aus dem Bereich der Operationsstelle und zum Abschalten des Lasergenerators (1), sobald die gemessenen Werte vorbestimmte Grenzwerte über- bzw. unterschreiten.

6. Vorrichtung nach mindestens einem der Ansprüche 1-6, wobei ein im Ultravioletten strahlender Excimer-Laser als Lasergenerator (1) vorgesehen ist und die dem Operationsfeld zugeführte Substanz im Ultravioletten absorbiert.

## Claims

1. Apparatus for performing surgery on biological tissue with
- an intermittently operated laser (1),
- means (3) for transmitting the laser radiation to the site of operation which means are arranged in a hand-piece (4, 14, 24, 44) so that its front end portion is directed to the side of operation,
- and an additional metering unit (9) for the supply of a substance to the site of operation which substance being absorbent in the wavelength region of the laser beam, characterized in that the apparatus further comprises
- a suction and irrigation unit (8) connected to the hand-piece (4, 14, 24, 44) for removing tissue ablated by the aid of the laser radiation, and
- an electronic unit which controles the supply of the absorbing substance by the metering unit (9) within the temporal intervals between the laser pulses.

2. Apparatus of claim 1, characterized in that the means for transmitting the laser radiation comprises a light conducting fibre (3) which is arranged in the hand-piece (4) and that the front end portion of the light conducting fibre (3) directed to the site of operation terminates at the tip (5) of the-hand-piece.

3. Apparatus of claim 2, characterized in that the light conducting fibre (3) for transmitting the laser radiation and the irrigation channel (46) of the suction and irrigation unit (8) are embedded into the handle of the hand-piece (4) and terminate at its end portion directed to the site of operation.

4. Apparatus of claim 2 or 3, characterized in that the end-face of the light conducting fibre (3) directed to the site of operation is rounded convexly so that it has focusing properties.

5. Apparatus of claim 1, characterized in that additionally a detector unit (7) is provided for measuring the radiation coming out of the site of operation and for switching off the laser (1) when the measured values exceed or drop below pregiven limit values.

6. Apparatus of one of the claims 1 to 6, wherein the laser (1) is an excimer laser emitting ultraviolet radiation and wherein the substance supplied to the site of operation is absorbing in the ultraviolet region.

## Revendications

1. Dispositif pour pratiquer la chirurgie sur un tissu biologique, comportant
- un générateur laser (1) activé de façon intermittente,
- des moyens (3) pour transmettre le rayonnement laser au champ opératoire qui sont logés dans une pièce à main (4,14,24,44) de sorte que leur face frontale est dirigée vers le champ opératoire,
- ainsi qu'une unité supplémentaire de dosage (9) pour l'envoi d'une substance, qui est absorbante dans la gamme des longueurs d'onde du faisceau laser, au champ opératoire,
caractérisé en ce que le dispositif contient en outre
- un dispositif d'aspiration et de balayage (8) pour éliminer le tissu retiré par le rayonnement laser, et qui est relié à la pièce a main (4,14,24,44), et
- une unité électronique, qui commande l'envoi de la substance absorbante, au moyen de l'unité de dosage (9), pendant l'intervalle de temps entre les impulsions laser.

2. Dispositif selon la revendication 1, caractérisé en ce qu'il est prévu, comme moyens pour transmettre le rayonnement laser, une fibre conductrice de lumière (3), qui est saisie par la pièce à main (4) et dans la pointe (5) de laquelle débouche la face frontale, tournée vers le champ opératoire, de la fibre conductrice de lumière (3).

3. Dispositif selon la revendication 2, caractérise en ce qu'une fibre conductrice de lumière (3), utilisée pour la transmission du rayonnement laser, et le canal de balayage (46) du dispositif d'aspiration et de balayage (8) sont enchâssés dans la poignée (44) de la pièce à main (44) et se terminent sur la face frontale de cette poignée, qui est dirigée vers le champ opératoire.

4. Dispositif selon la revendication 2 ou 3, caractérisé en ce que la face frontale, tournée vers le champ opératoire, de la fibre conductrice de lumière (3) est enroulée avec une forme convexe de sorte qu'elle possède des caractéristiques de focalisation.

5. Dispositif selon la revendication 1, caractérisé en ce qu'il est en outre prévu une unité de détection (7) qui sert à mesurer le rayonnement sortant de la zone du champ opératoire et à arrêter le générateur laser (1) des que les valeurs mesurées dépassent, par valeurs supérieures ou inférieures, des valeurs limites prédéterminées.

6. Dispositif selon au moins l'une des revendications 1-6, dans lequel il est prévu, comme générateur laser (1), un laser excimère émettant dans l'ultraviolet et que la substance envoyée au champ opératoire est absorbée dans l'ultraviolet.
